# EUROPEAN PATENT SPECIFICATION

(11) **EP 1 685 804 B1**
(45) Date of publication and mention of the grant of the patent: **31.10.2007**
(21) Application number: 06100921.3
(22) Date of filing: 27.01.2006
(51) Int. Cl.: A61B 17/80, A61L 31/14, A61B 17/86, A61B 17/00

(54) **System for stabilizing spine**
System zur Stabilisierung der Wirbelsäule
Système pour stabiliser la colonne vertébrale

(30) Priority: 01.02.2005 US 48109
(43) Date of publication of application: 02.08.2006
(73) Proprietor: Inion Oy, 33520 Tampere (FI)
(72) Inventor: HAPPONEN, Harri, 33820, TAMPERE (FI); VUORISALO, Vesa, 33530, TAMPERE (FI)
(74) Representative: Kaukonen, Juha Veikko

(56) References cited:
- WO-A-20/05039426
- FR-A- 2 739 151
- FR-A- 2 845 588
- US-A1- 2004 260 291
- US-B1- 6 342 057
- US-B1- 6 350 265
- US-B1- 6 605 090

## Description

### BACKGROUND OF THE INVENTION

The invention relates to a system for stabilizing the spine, the system comprising:a fixation screw with a threaded section, a fixation plate with a top and bottom surface, and a threading tool, wherein the bottom surface is to be arranged against the bone tissue and the fixation plate has a pre-drill hole which is arranged on the side of said top surface and by means of which the actual fixation hole extending through the plate from the top surface to the bottom surface will be made, the threading tool configured to form a uniform thread to the fixation hole and to the bone tissue, and the fixation screw configured to fit into the threaded fixation hole and the threaded the bone tissue so as to lock the screw to the fixation plate.

Various injuries may be caused to the spine due to degeneration, tumors, and fractures and dislocations caused by physical trauma. In some operations directed to the spine, fixation plates are used that are fastened to the bone tissue of the spine by means of fixation screws arranged through fixation holes in the fixation plate. Cervical spine operations are made either anteriorily or posteriorily depending on the injury to be treated. A typical application of the fixation plate is the anterior ossification of cervical vertebrae. A damaged disc is removed from between the vertebrae and replaced for instance by a bone implant taken from the iliac to keep the intervertebral space intact. The vertebrae above and below the bone implant are fastened together with a fixation plate. The fixation plate is fastened to the vertebrae by screws. The fixation plate supports the vertebrae and prevents the bone implant from sliding away from between the vertebrae.

A method applicable to repairing damage to the spine and the fixation plates and screws used therein is disclosed in US patent 5,601,553. A first fixation hole of the fixation plate described therein is perpendicular to a first plane of the top surface of the plate and, correspondingly, a second fixation hole is perpendicular to a second plane of the top surface of the plate. The first and second planes of the top surface intersect, whereby the axes of the fixation holes also intersect.

In this solution, as in other prior art arrangements, it may be problematic to arrange the fixation screws in such a manner that the fixation plate fastens appropriately to the bone tissue.

US 2004/0260291, upon which the two part from of claim 1 is based, teaches a bone plate system including a plate and fixation devices, such as screws. The plate can be made of bioresorbable materials. The plate can comprise one or more tappable apertures. One or more of these apertures can be selected to be tapped, and the angles at which the apertures are tapped can be selected by the operator. The tapping can be done *in situ.* FR 2 845 588 discloses a self-locking osteosynthesis device comprising a plate which has holes for fixation screws. The specialty of the device is that at least in the areas defining the screw holes, the plate is made from a material having mechanical properties such that the periphery of said holes can be self-tapped by tapping screws which can be used to fix the plate. The angles at which the screws are tapped can be selected by the operator.

### BRIEF DESCRIPTION OF THE INVENTION

According to the invention presented above, a novel solution is obtained for the above-mentioned problem.

The system for stabilizing the spine of the invention is characterized in that the pre-drill hole is a blind hole extending to a distance from the bottom surface of the fixation plate.

The invention provides the advantage that the fixation screw can be arranged to such a position relative to the fixation plate that the fixation screw and thus also the fixation plate can be fixed to the bone tissue in the best possible manner taking into consideration medical and operational criteria.

An idea of the invention is that the pre-drill hole is a blind hole that extends to a distance from the bottom surface of the plate. An advantage is that the making of the fixation hole is then essentially as easy in any direction.

An idea of an embodiment of the invention is that the pre-drill hole form a countersink and that the head of the fixation screw is designed with respect to the countersink in such a manner that it does not form an essential protuberance above the plane of the top surface of the fixation plate. This provides the advantage that the height of the system can be kept as small as possible, which means that it disturbs the neighboring tissue as little as possible.

An idea of an embodiment of the invention is that the thread of the threaded section of the fixation screw is arranged to be changeable so that the height of the screw ridge is greater in the section closer to the distal end than in the section further away from the distal end. This provides the advantage that a high ridge fastens optimally to the cancellous bone, whereas a low ridge fastens optimally to cortical bone.

### BRIEF DESCRIPTION OF THE FIGURES

The invention is described in greater detail in the attached drawings, in which

Figures 1a to 1c are schematic representations of a fixation plate not belonging to the arrangement of the invention,

Figures 2a and 2b are schematic representations of a fixation screw belonging to the arrangement of the invention,

Figure 3 is a schematic representation of a fixation plate not belonging to an arrangement of the invention as seen from the end and with the fixation plate in cross-section,

Figures 4a to 4c are schematic representations of a second fixation plate belonging to the arrangement of the invention,

Figures 5a and 5b are schematic representations of a second fixation screw belonging to the arrangement of the invention,

Figure 6 is a schematic representation of a second arrangement of the invention as seen from the end and with the fixation plate in cross-section, and

Figures 7a to 7c are schematic representations of a third fixation plate belonging to the arrangement of the invention.

In the figures, the invention is shown simplified for the sake of clarity. Similar parts are marked with the same reference numbers in the figures.

### DETAILED DESCRIPTION OF CERTAIN PREFERRED EMBODIMENTS

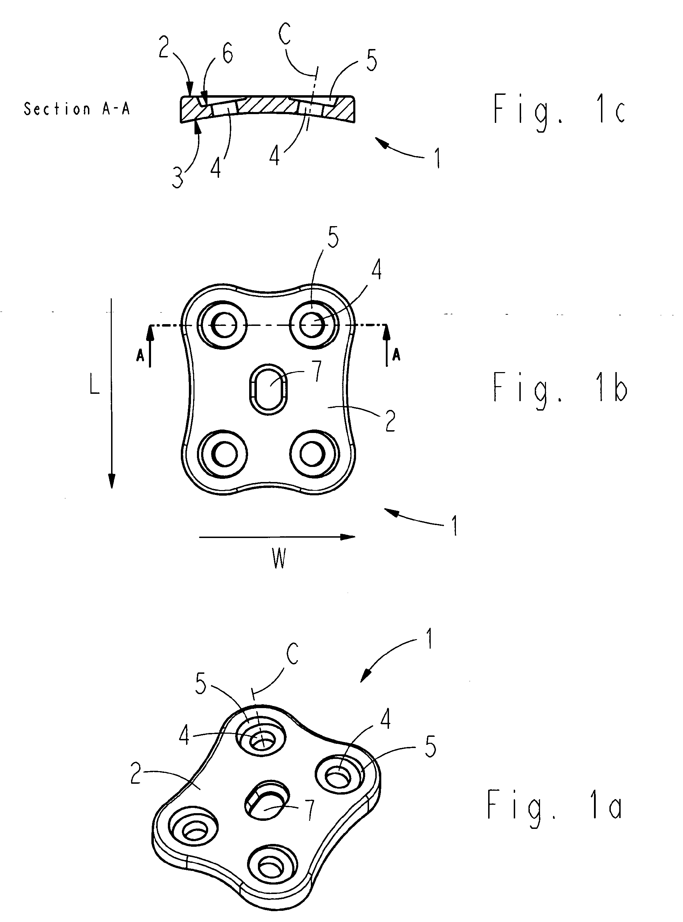

Figure 1a is a schematic representation of a fixation plate in perspective, Figure 1b shows the same fixation plate from the direction of the top surface, and Figure 1c shows it from the side in cross-section along section A-A. It should be noted that the fixation plate is in later on referred to as 'plate'.

The plate 1 is preferably made of a biodegradable polymer material absorbing into the organ system that is prepared by polymerising or copolymerising for instance lactic acid, L-lactide, D-lactide, D,L-lactide, mesolactide, glycolic acid, glycolide or a cyclic ester copolymerised with lactide, or of any other corresponding material known per se to a person skilled in the art, which will not be discussed in this context in greater detail. Other suitable biodegradable polymers, copolymers and polymer mixtures are listed in the following publications, for instance:
- "Encyclopedic Handbook of Biomaterials and Bioengineering, Part A," Donald, L. Wise, Debra J. Trantolo, David E. Altobelli, Michael J. Yaszemski, Joseph D. Gresser, Edith R. Schwartz, 1992, by Marcel Dekker, Inc., pages 977 to 1007,
- "Biodegradable fracture-fixation devices in maxillofacial surgery," R. Suuronen, Int. J. Oral Maxillofac. Surg., 1993, 22: 50 to 57,
- "Critical Concepts of Absorbable Internal Fixation," William S. Pietrzak, Portland Bone Symposium, Portland, Oregon, August 4 to 7, 1999,
- *"*High-impact poly(UD-lactide) for fracture fixation: in vitro degradation and animal pilot study," Jan Tams, Cornelis A.P. Joziasse, Ruud R.M. Bos, Fred R. Rozema, Dirk W. Grijpma and Albert J. Pennings, Biomaterilas 1995, Vol. 16, No. 18, pages 1409 to 1415,
- *"*A Review of Material Properties of Biodegradable and Bioresorbable Polymers and Devices for GTR and GBR Applications," Dietmar Hutmacher, Markus B. Hürzeler, Henning Schliephake, The International-Journal of Oral & Maxillofacial Implants, Volume 11, Number 5, 1996, pages 667 to 678, and
- *"*Orthopaedic Application for PLA-Pga Biodegradable Polymers," Kyriacos A. Athanasiou, Mauli Agrawal, Alan Barber, Stephen S. Burkkhart, The Journal of Arthroscopic and Related Surgery, Vol. 14, No. 7 (October), 1988: 726 to 737.

Further, it is obvious to a person skilled in the art that the material can be a composite that contains two or more materials or monomers, polymer chains, having the essential property of dissolving in the system. A composite can contain bio-glass, bio-ceramics, biologically active components, a pharmaceutical, such as antibiotic or growth factor, or the like.

Further, the material may comprise softeners, such as a pyrrolidone plasticizer. The pyrrolidones useful in the implants or methods of the present invention are any pyrrolidones known in the art of chemistry to have plasticizing properties without having tissue impairing effects or toxic effects. Such pyrrolidones include alkyl- or cycloalkyl-substituted pyrrolidones, such as N-methyl-2-pyrrolidone (NMP), 1-ethyl-2-pyrrolidone (NEP), 2-pyrrolidone (PB), and 1-cyclohexyl-2-pyrrolidone (CP), with NMP and NEP being preferred examples.

When examining the structure of the plate, it is detected that it has a top surface 2 and a bottom surface 3. The bottom surface 3 is intended to be arranged against the bone tissue to be stabilized and, correspondingly, the top surface 2 away from it. In the plates 1 shown in the figures, the top surface 2 has a substantially straight profile. The cross-section of the bottom surface 3 is, however, curved so that the bottom surface 3 curves with a straight line parallel to the longitudinal direction L as the curvature axis of the plate 1. The shape of the surfaces 2, 3 and other shapes of the plate 1 can naturally differ from the shapes shown in the figures.

A pre-drill hole 4 is formed on the top surface 2 of the plate and, in this case, it is a rotationally symmetrical and cylindrical through-hole extending from the top surface 2 to the bottom surface 3 and its imaginary longitudinal center axis is marked by reference mark C. The plate 1 has four pre-drill holes 4 in total, but their number may vary.

A countersink 5 is formed at the mouth of the pre-drill hole 4. The bottom plane 6 of the countersink 5 is substantially perpendicular to the center axis C; in other words, the center axis C is the diagonal of the spatial plane in the direction of the bottom plane 6.

The plate 1 also comprises a guide hole 7 arranged at the symmetrical center point of the plate 1. In the embodiment shown in the figures, the guide hole 7 is an oval through-hole extending through the plate 1, to which an instrument can be detachably attached to assist in the handling of the typically small plate 1 during its installation. Such instruments are known per se, so they are not discussed in more detail herein. It is also clear that the guide hole 7 can also be shaped and placed otherwise, as is the fact that the plate 1 can also be implemented without the guide hole 7.

The plate 1 is fastened in place relative to the bone tissue in such a manner that, first, it is arranged correctly relative to the bone tissue. This step may include shaping steps of the plate, in which the plate 1 is bent or its shape modified in some other manner known per se so as to fit it appropriately on the surface of the bone tissue. After this, through the pre-drill hole 4 but not necessarily in the direction of its center axis C, a hole is drilled in the bone tissue under the plate 1. The hole drilled in the bone tissue and the actual fixation hole drilled through the pre-drill hole 4 to the plate 1 are threaded with a threading tool in such a manner that a uniform thread is made to them. After this, a fixation screw is tightened into the threaded hole - which thus is first formed of the threaded section in the plate 1 and then of the threaded section in the bone tissue. The making of the fixation hole and its fastening to the bone tissue are described later in more detail.

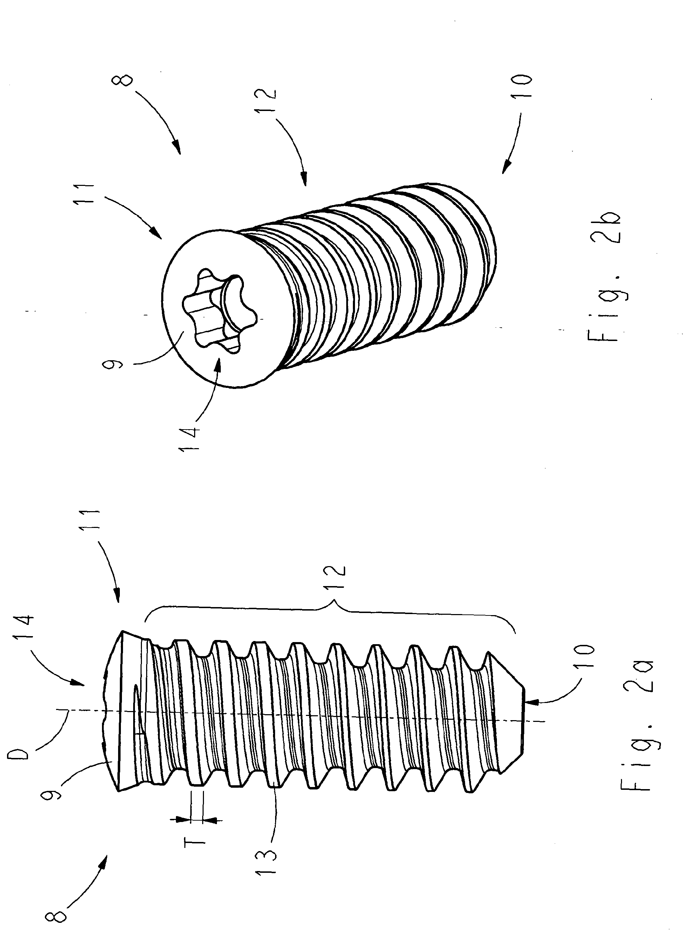

Figure 2a is a schematic side view of a fixation screw belonging to the arrangement of the invention, and Figure 2b is a perspective view from the direction of the proximal end. It should be noted that the fixation screw is later referred to as 'screw'. The screw 8 can be made, for example, of the materials specified earlier as the manufacturing materials for the plate.

The screw 8 has a proximal end 9 and a distal end 10. A head 11 of the screw, the maximum diameter of which is at the same time the maximum diameter of the screw 8, is arranged at the proximal end 9. The ratio of the screw head 11 diameter to the outer diameter of the threaded section 12 is preferably 10:9-10:7.

A threaded section 12 is formed between the head 11 and the distal end 10. The threaded section of the screw shown in Figures 2a, 2b has one ridge 13 extending as a uniform thread from the head 11 to the distal end 10. The outer diameter of the threaded section 12 is constant with the exception of the convergent area of the top cone close to the distal end 10. The height of the ridge 13 is, however, smaller close to the proximal end 9 than to the distal end 10. In addition, the thickness T of the ridge 13 is greater close to the proximal end 9 than to the distal end 10 and in the section close to the proximal end 9.

The threaded section 12 may also have two or more ridges 13, in which case it is a multi-end threaded section. The ridge 13 can be discontinuous in that, at one point, it is completely or partially cut. In addition, the profile, height and thickness of the ridge 13 may be constant substantially throughout the length of the threaded section. The pitch of the threaded section 12 may also be different at different parts thereof.

The screw 8 has an imaginary longitudinal center axis D, around which the screw 8 rotates when the threaded section 12 is tightened to its counter-threads.

A counterpart 14 is arranged to the proximal end 9 for a tool required to tighten the screw. It should be noted that the tool is not shown in the figure. In its presented embodiment, the counterpart 14 resembles a Torx® driver, but it can naturally also be shaped otherwise.

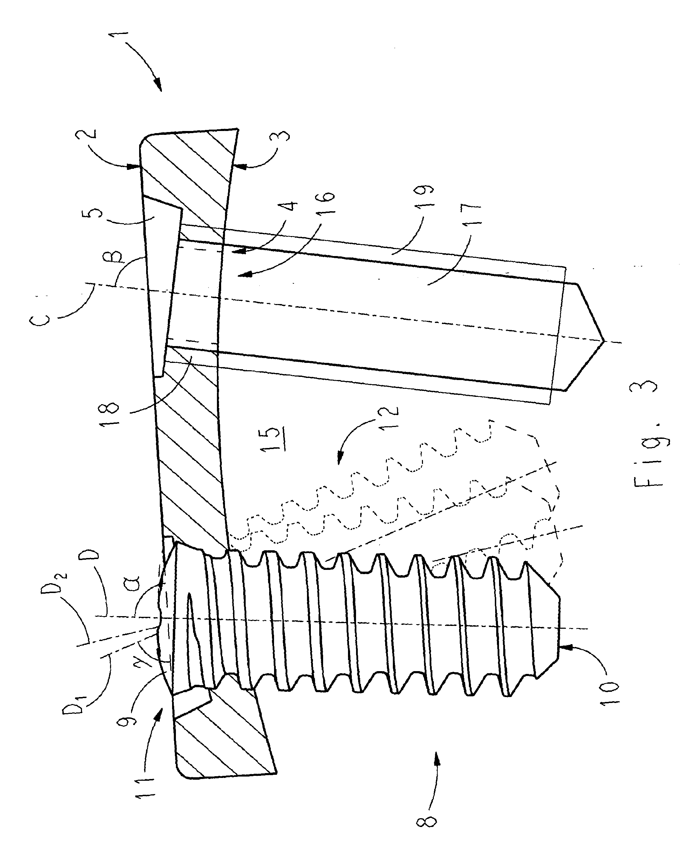

Figure 3 is a schematic representation of an arrangement of the invention comprising the plate 1 shown in Figures 1a to 1c and the screws 8 shown in Figures 2a, 2b as seen from the end and with the plate 1 in cross-section.

Actual fixation holes 16 are made into the plate 1. The fixation holes 16 are made using the pre-drill holes 4 - drawn by dashed lines to the rightmost fixation hole 16 only in Figure 3 - and the threaded sections 18 of the fixation holes are made to them.

A bone tissue screw hole 17 is made to the bone tissue 15 and a threaded section 19 of the bone tissue is made to it. The bone tissue screw hole 17 and the fixation hole 16 are parallel and coaxial with each other, and they are made by preferably drilling them at the same time. Drilling the holes at the same time ensures that the holes 16, 17 are parallel and coaxial.

The threaded section 18 of the fixation hole and the threaded section 19 of the bone tissue are made with the same tool and during the same step in such a manner that the threaded sections 18, 19 form a continuous threaded section from the plate 1 to the bone tissue 15. The manufacturing material of the plate 1 allows the making of the fixation hole 16 and its threaded section 18 with for instance any tools used in making bone holes. Bone drills and screw taps known per se can be used in making the fixation holes 16, 17 and their threaded sections.

The plate 1 can be fastened to the bone tissue 15 for instance as follows.

An appropriately shaped plate 1 is arranged to its fastening point relative to the bone tissue 15. The instrument attached detachably to the guide hole 7 can be used herein.

Next, a hole of the required size and depth is drilled *in situ* through the pre-drill hole 4 in the plate to the bone tissue 15. The hole forms the fixation hole 16 and the bone tissue screw hole 17. Not only the above-mentioned instrument, but also a drill guide can be used in this to guide the drill at the correct angle to the plate 1 and to also protect the surrounding tissue from the revolving drill bit.

After this, threads are made to the hole with a screw tap or some other threading tool. The result is a thread that is formed of the threaded section 18 of the fixation hole in the plate 1 and the threaded section 19 in the bone tissue. A drill guide can be used to protect the surrounding tissue from the revolving threading tool.

Another alternative is to use a combination of a drill and screw tap, i.e. a self-tapping drill. This has a drilling tip and a threading section. In other words, the hole and its threading is done at the same time with the same revolving movement of the self-tapping drill. One such self-tapping drill is disclosed in US patent application 2004/0092950.

It is also possible to use a compressing drill or a screw tap that does not remove bone, but compresses it tightly around the drill hole. It is naturally also possible to use a compressing or partially compressing combination of a drill and a screw tap.

The pre-drill hole 4 facilitates the fastening of the plate 1 to the bone tissue 15, because it forms a clear starting point on the top surface 2 side of the plate, from which the making of the fixation hole 16 can be started.

The fixation holes 16 are arranged at a first angle relative to the top surface 2 of the plate 1; in other words, the longitudinal center axis C of the fixation hole 16 and the center axis D of the screw 8 arranged therein form a first angle relative to the top surface 2 of the plate 1. In the embodiment shown in Figures 2a, 2b, the first angles of the left and right fixation holes are unequal with respect to each other: in the case of the left fixation hole 16, the size of the first angle is α and, in the case of the right fixation hole 16, the size of the first angle is β.

The size of the first angle and, thus, the angle of the center axis C of the fixation hole 16 and the longitudinal center axis D of the screw 8 arranged therein relative to the plate 1 can be selected case by case in the most appropriate manner. In connection with the left fixation hole 16, alternative positions for the longitudinal center axis D of the screw 8 are shown by dashed lines and marked by reference markings D₁ and D₂. For instance, if for the criteria set by the fastening of the plate 1 and the stabilization of the bone tissue 15, it were best that the center axis of the left screw 8 was in the position shown by reference marking D₁, i.e. at angle γ relative to the plate 1, a fixation hole 16 would be made using the left pre-drill hole with the first angle of the center axis C being the above-mentioned γ.

When the screw 8 is tightened into the fixation hole 16 and on to the screw hole 17 of the bone tissue, it tightens to the threads 18, 19 therein in such a manner that a rigid constraint is formed between it and the plate to support the fixation, even though the screw 8 did loosen slightly in the bone. A rigid constraint means that a substantial micro-movement or angle movement relative to the plate 1 does not take place in the screw 8. Due to the low and thick ridge 13 close to the head 11 of the screw, the screw 8 locks very well to the thread in the plate 1.

The size of the first angle can be selected to be within specific limit values. The maximum value of the first angle can be for instance within the range of 70° to 90°, preferably 75° to 85°. The first angle can be divided into two orthogonal components that are its projections in the longitudinal direction L and lateral direction W of the plate. In other words, the center axis C, D can form an angle differing from right-angle both in the longitudinal direction L and lateral direction W of the fixation plate. Thus, the center axis C, D can be tilted both in the longitudinal direction L and lateral direction W of the plate to obtain the best possible angle for the fixation hole 16 and to the screw 8. It should be noted that the longitudinal direction L and lateral direction W of the plate 1 are shown in Figures 1 b, 4b, and 7b. The screws 8 are preferably not arranged parallel into the bone tissue 15, because screws 8 oriented in different directions lock the plate 1 tighter to the bone tissue 15 and increase the strength required to pull out the screws 8.

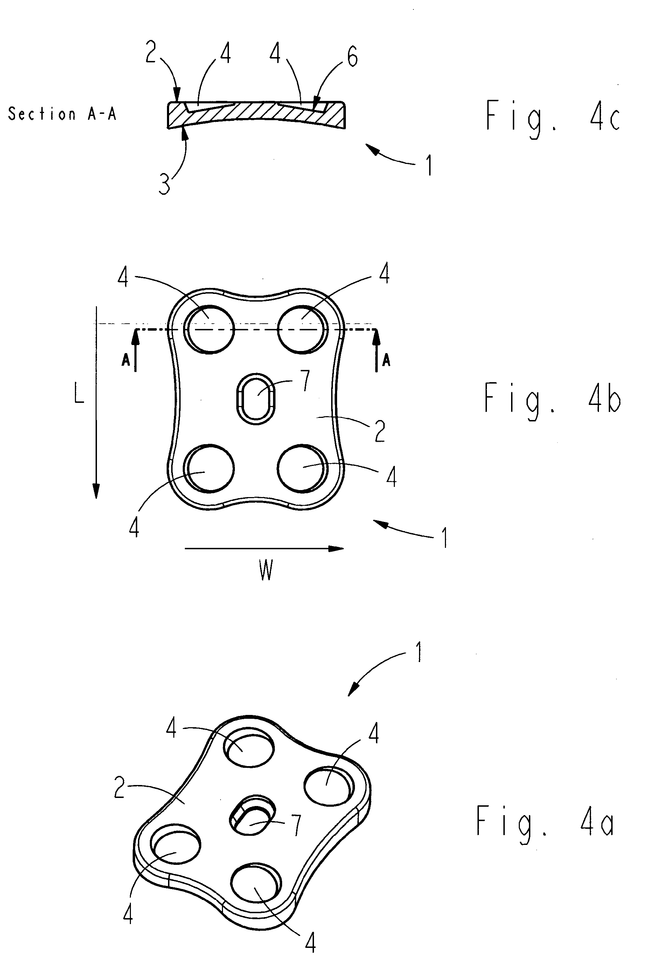

Figure 4a is a schematic perspective view of a fixation plate belonging to a first arrangement of the invention, Figure 4b shows the same fixation plate as seen from the top surface, and Figure 4c from the side in cross-section along section A-A.

In this case, the plate 1 does not have a pre-drill hole through it, but the pre-drill hole 4 is a blind hole formed on the top surface 2 of the plate and extends to a distance from the bottom surface 3 of the plate. The basic form of the pre-drill hole 4 shown in Figures 4a to 4c is a cut circular cone with its center axis and bottom 6 arranged at an oblique angle relative to the top surface of the plate. Otherwise the plate 1 is mainly similar to the plate 1 shown in Figures 1a to 1c.

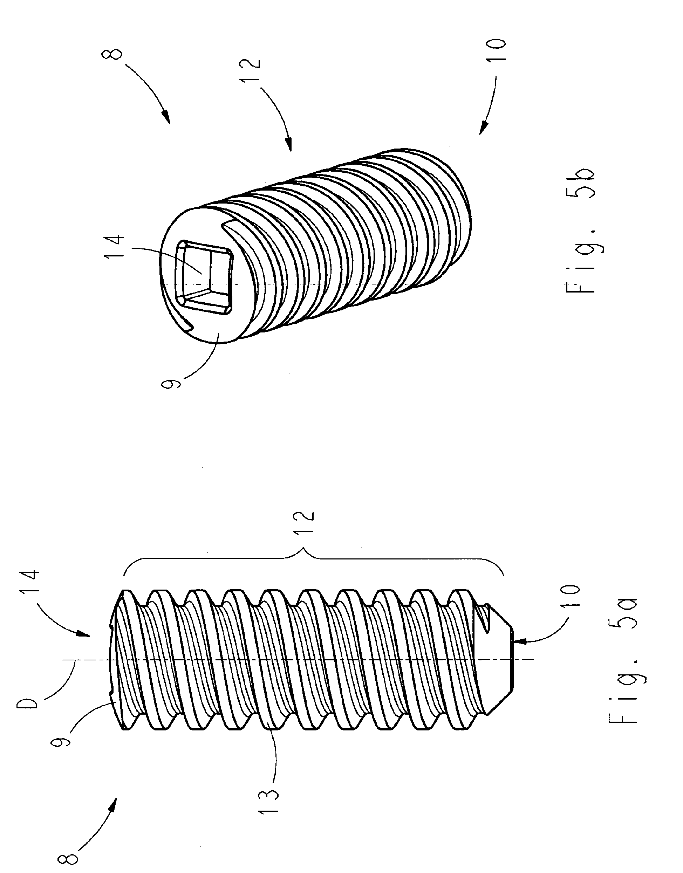

Figure 5a is a schematic side view of a second fixation screw belonging to the arrangement of the invention and Figure 5b is a perspective view thereof from the direction of the proximal end.

The screw 8 shown herein is a headless screw. The threaded section 12 of the screw 8 extends from the proximal end 9 to the distal end 10 and, thus, substantially along the entire length of the screw 8.

The threaded section 12 has a double-ended ridge 13, the height of which is constant substantially along the entire length of the threaded section 12. The thickness of the ridge 13 is also constant substantially along the entire length of the threaded section.

The screw 8 can be tightened into the fixation hole into such a depth that the proximal end 9 is level with the mouth of the fixation hole or even inside the fixation hole. The screw 8 then does not form a protrusion from the plate 1, which might have a disadvantageous impact on the tissues surrounding the arrangement.

A counterpart 14 with a square cross-section for a tool is arranged to the proximal end 9. The screw 8 can be tightened around its longitudinal center axis D with a tool arranged into the counterpart 14.

The screw 8 can be cannulated, i.e. a channel extending from the proximal end 9 to the distal end 10 and parallel to the center axis D can pierce it.

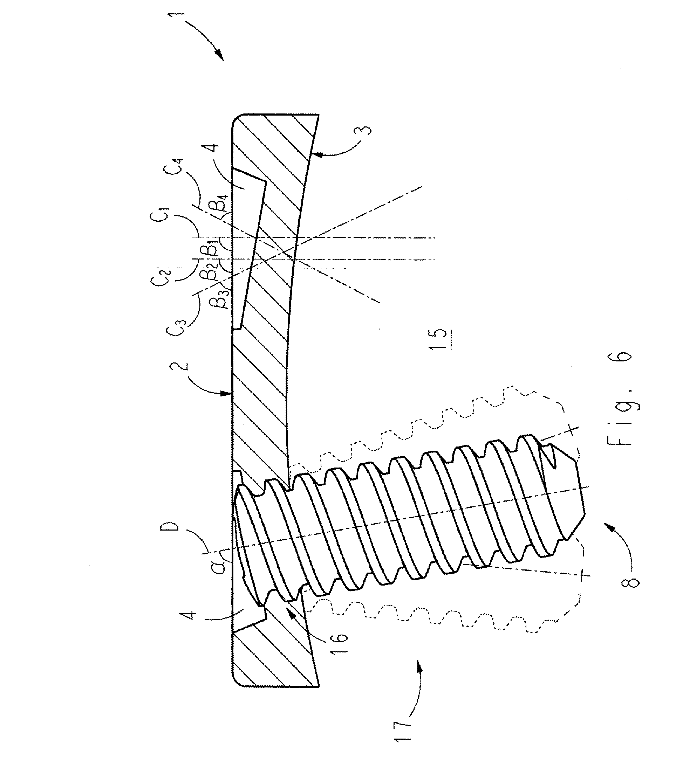

Figure 6 is a schematic side view of the first arrangement of the invention that comprises the fixation plate shown in Figures 4a to 4c and the screws shown in Figures 5a, 5b, with the fixation plate in cross-section.

A fixation hole 16 and a bone tissue screw hole 17 and their uniform threaded sections are drilled *in situ* through the left pre-drill hole 4. A screw 8 is tightened into the threaded sections to fasten the plate 1 to the bone tissue 15. The longitudinal center axis C of the fixation hole 16 and thus also the longitudinal center axis D of the screw 8 are at a first angle relative to the top surface 2 of the plate. Herein, the size of the first angle is α. The fixation hole 16 could also have been made such that the first angle of the screw 8 tightened therein was other than α. Therefore, the figure shows two of such alternatives with dashed lines.

The left pre-drill hole 4 forms a countersink for the screw 8 on the top surface 2 of the plate. Due to the countersink, the proximal end 9 of the screw 8 tightened into the threaded section 18 of the fixation hole settles into the plate 1 so that is does not extend above the level of the top surface 2. Because of this, the total height of the system on the surface of the bone tissue 15 is very small.

In the situation shown in Figure 6, a fixation hole has not yet been made through the right pre-drill hole 4. The figure shows a few alternatives for the position of the fixation hole to be made through the right pre-drill hole 4 as shown by means of the center axes C₁ to C₄ of the fixation holes.

When selecting the correct position of the fixation hole 16 - and the screw 8 to be tightened therein - relative to the plate 1, it is possible to vary not only the angle of the fixation hole 16 relative to the plate 1 - i.e. the first angle α, β₁ to β₄ - but also the location where the mouth of the fixation hole 16 is made in the pre-drill hole 4. The mouth of the fixation hole 16 need not be arranged in the middle of the pre-drill hole 4, i.e. symmetrically to the pre-drill hole, but it can be arranged to the side in the longitudinal direction L and/or lateral direction W of the plate, i.e. asymmetrically to the pre-drill hole 4. The surgeon fastening the plate 1 has a great deal of choice in arranging each screw 8 to be arranged into the plate 1 individually in exactly the correct position relative to the plate 1 and bone tissue 15.

After the position and direction of the fixation hole to be made through the right pre-drill hole 4 and the bone tissue 15 screw hole to be made simultaneously in connection with it are selected, the fixation hole and bone tissue screw hole are made by drilling through the plate 1 to the bone tissue 15 a hole having the required diameter and length. Threads are made to this hole by using a threading tool known per se. The hole and threads can naturally also be made with a self-tapping drill. After making the threads, certain hole-finishing measures can be taken, after which a screw is tightened into the threads.

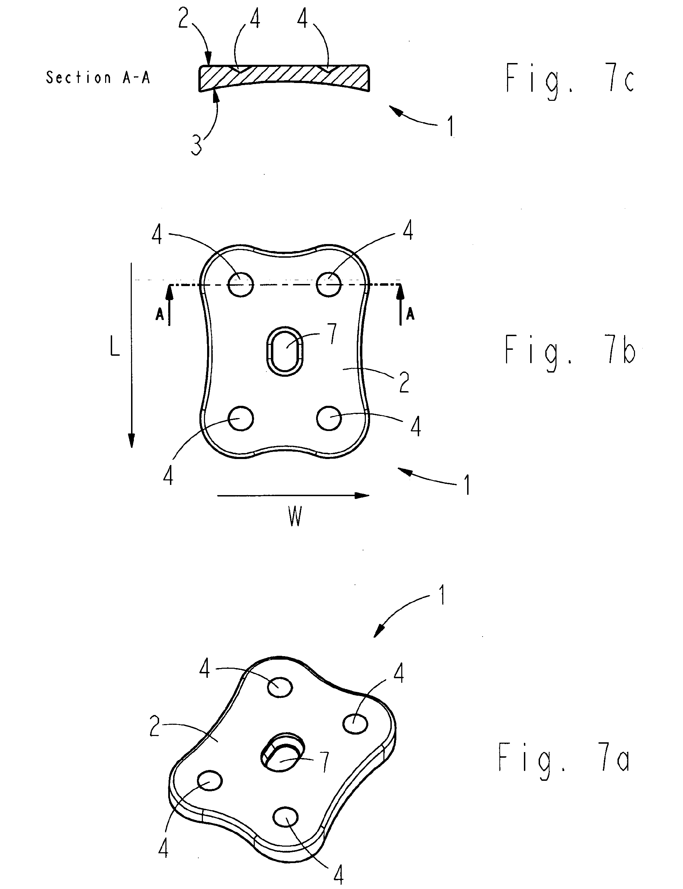

Figures 7a to 7c are schematic representations of a second fixation plate belonging to the arrangement of the invention. Like the fixation plates described above, this too can be made from a material that dissolves in the organ system.

The plate 1 comprises four pre-drill holes 4 arranged on the side of the top surface 2. The pre-drill hole 4 is now a blind hole in the shape of a circular cone and its tip forms the bottom of the pre-drill hole 4. The pre-drill hole 4 can also have some other shape than that shown in the figures: it can for instance be a tri- or multi-angular cone, a recess in the shape of a spherical surface, etc. The actual fixation hole and the required threaded sections are made as described in the preceding figures.

The drawings and the related description are only intended to illustrate the idea of the invention. The invention may vary in detail within the scope of the claims.

## Claims

1. A system for stabilizing the spine, the system comprising:
a fixation screw (8) with a threaded section (12),
a fixation plate (1) with a top and bottom surface (2, 3), and a threading tool,
wherein the bottom surface (3) is to be arranged against the bone tissue (15) and the fixation plate (1) has a pre-drill hole (4) which is arranged on the side of said top surface (2) and by means of which the actual fixation hole (16) extending through the plate from the top surface (2) to the bottom surface (3) will be made,
the threading tool configured to form a uniform thread to the fixation hole (16) and to the bone tissue, and the fixation screw (8) configured to fit into the threaded fixation hole (18) and the threaded bone tissue (19) so as to lock the screw (8) to the fixation plate (1), **characterized in that** the pre-drill hole (4) is a blind hole extending to a distance from the bottom surface (3) of the fixation plate (1).

2. A system as claimed in claim 1, wherein the fixation plate (1) and screw (8) are made of a biodegradable material.

3. A system as claimed in any one of claims 1 to 2, wherein the fixation plate (1) comprises a guide hole (7), to which a guide tool facilitating the handling of the fixation plate (1) can be arranged.

4. A system as claimed in claim 3, wherein the guide hole (7) is arranged at the symmetrical center point of the fixation plate (1).

5. A system as claimed in any one of claims 1 to 4, wherein the pre-drill hole (4) of the fixation hole forms a countersink of the fixation hole (16).

6. A system as claimed in any one of claims 1 to 5, wherein the mouth of the threaded fixation hole (18) can be located asymmetrically in relation to the pre-drill hole (4).

7. A system as claimed in any one of claims 1 to 6, wherein the fixation screw (8) comprises a proximal end (9) with a counterpart for a tool arranged thereto.

8. A system as claimed in claim 7, wherein a screw head (11) having a larger diameter than the maximum outer diameter of the threaded section (12) is arranged at the proximal end (9).

9. A system as claimed in claim 8, wherein the ratio of the screw head (11) diameter to the outer diameter of the threaded section (12) is 10:9 to 10:7.

10. A system as claimed in any one of claims 7 or 8, wherein the pre-drill hole (4) forms a countersink (5) and the head (11) of the fixation screw is designed relative to the pre-drill hole (4) in such a manner that it fits below the top surface level of the plate.

11. A system as claimed in any one of claims 1 to 7, wherein the fixation screw (8) is a headless screw and the threaded section of the screw (12) is arranged to extend to the proximal end (9).

12. A system as claimed in any one of claims 1 to 11, wherein the thread of the threaded section (12) of the fixation screw is arranged to change in such a manner that the height of the screw ridge (13) is greater in the section close to the distal end (10) than in the section further away from the distal end.

13. A system as claimed in any one of claims 1 to 13, wherein the angle of the threaded fixation hole (18) can be chosen to form an angle differing from right-angle both in the longitudinal (L) and lateral (W) direction of the fixation plate (1).

## Patentansprüche

1. System zur Stabilisierung der Wirbelsäule, das System umfasst:
eine Fixationsschraube (8) mit einem Gewindeabschnitt (12),
eine Fixationsplatte (1) mit einer oberen und einer unteren Fläche (2, 3) und ein Gewindewerkzeug,
wobei die untere Fläche (3) an dem Knochengewebe (15) anzuordnen ist und die Fixationsplatte (1) eine Vorbohrung (4) aufweist, die auf der Seite der oberen Fläche (2) angeordnet ist und mittels derer das tatsächliche Fixationsloch (16), das sich durch die Platte von der oberen Fläche (2) zu der unteren Fläche (3) erstreckt, geschaffen wird, und
wobei das Gewindewerkzeug ausgelegt ist, um in dem Fixationsloch (16) und in dem Knochengewebe ein einheitliches Gewinde auszubilden, und die Fixationsschraube (8) ausgelegt ist, so in das Gewindefixationsloch (18) und das Gewindeknochengewebe (19) eingefügt zu sein, um die Schraube (8) an der Fixationsplatte (1) zu arretieren, und ist **dadurch gekennzeichnet, dass** die Vorbohrung (4) ein Blindloch ist, das sich in einem Abstand von der unteren Fläche (3) der Fixationsplatte (1) erstreckt.

2. System nach Anspruch 1, wobei die Fixationsplatte (1) und die Schraube (8) aus biologisch abbaubarem Material gefertigt sind.

3. System nach einem der Ansprüche 1 und 2, wobei die Fixationsplatte (1) ein Führungsloch (7) umfasst, an dem ein Führungswerkzeug eingerichtet werden kann, das die Handhabung der Fixationsplatte (1) erleichtert.

4. System nach Anspruch 3, wobei das Führungsloch (7) auf dem symmetrischen Mittelpunkt der Fixationsplatte (1) angeordnet ist.

5. System nach einem der Ansprüche 1 bis 4, wobei die Vorbohrung (4) des Fixationslochs eine Versenkung des Fixationslochs (16) bildet.

6. System nach einem der Ansprüche 1 bis 5, wobei der Eingang des Gewindefixationslochs (18) in Bezug auf die Vorbohrung (4) asymmetrisch angeordnet sein kann.

7. System nach einem der Ansprüche 1 bis 6, wobei die Fixationsschraube (8) ein proximales Ende (9) mit einem daran angeordneten Gegenstück für ein Werkzeug umfasst.

8. System nach Anspruch 7, wobei an dem proximalen Ende (9) ein Schraubenkopf (11) mit einem größeren Durchmesser als der maximale Außendurchmesser des Gewindeabschnitts (12) angeordnet ist.

9. System nach Anspruch 8, wobei das Verhältnis des Durchmessers des Schraubenkopfes (11) zu dem Außendurchmesser des Gewindeabschnitts (12) 10:9 bis 10:7 ist.

10. System nach einem der Ansprüche 7 oder 8, wobei die Vorbohrung (4) eine Versenkung (5) bildet und der Kopf (11) der Fixationsschraube relativ zu der Vorbohrung (4) derartig ausgelegt ist, dass er unterhalb der oberen Flächenebene der Platte eingefügt ist.

11. System nach einem der Ansprüche 1 bis 7, wobei die Fixationsschraube (8) ein Gewindestift ist und der Gewindeabschnitt der Schraube (12) eingerichtet ist, um sich zu dem proximalen Ende (9) zu erstrecken.

12. System nach einem der Ansprüche 1 bis 11, wobei der Gewindegang des Gewindeabschnitts (12) der Fixationsschraube so eingerichtet ist, dass er sich derartig ändert, dass die Höhe des Schraubengrates (13) in dem Abschnitt nahe dem distalen Ende (10) größer ist als in dem Abschnitt weiter von dem distalen Ende entfernt.

13. System nach einem der Ansprüche 1 bis 12, wobei der Winkel des Gewindefixationslochs (18) so gewählt werden kann, um einen Winkel zu bilden, der von einem rechten Winkel sowohl in der Längsrichtung (L) als auch in der Seitenrichtung (W) der Fixationsplatte (1) abweicht.

## Revendications

1. Système permettant de stabiliser la colonne vertébrale, le système comprenant :
■ une vis de fixation (8) ayant une section filetée (12),
■ une plaque de fixation (1) ayant une face supérieure et une face inférieure (2, 3), et un outil à fileter,
dans lequel la face inférieure (3) est destinée à être disposée contre le tissu osseux (15) et la plaque de fixation (1) présente un avant-trou (4) qui est disposé du côté de ladite face supérieure (2) et au moyen duquel le véritable trou de fixation (16) se prolongeant à travers la plaque depuis la face supérieure (2) vers la face inférieure (3) sera pratiqué,
l'outil à fileter est configuré pour conférer un filetage uniforme au trou de fixation (16) et au tissu osseux, et la vis de fixation (8) est configurée pour loger dans le trou de fixation fileté (18) et dans le tissu osseux fileté (19) afin de verrouiller la vis (8) sur la plaque de fixation (1), **caractérisé en ce que** l'avant-trou (4) est un trou borgne se prolongeant jusqu'à une certaine distance de la face inférieure (3) de la plaque de fixation (1).

2. Système selon la revendication 1, dans lequel la plaque de fixation (1) et la vis (8) sont en matériau biodégradable.

3. Système selon l'une quelconque des revendications 1 à 2, dans lequel la plaque de fixation (1) comprend un trou de guidage (7), sur lequel un outil de guidage facilitant la manipulation de la plaque de fixation (1) peut être adapté.

4. Système selon la revendication 3, dans lequel le trou de guidage (7) se trouve au centre de symétrie de la plaque de fixation (1).

5. Système selon l'une quelconque des revendications 1 à 4, dans lequel l'avant-trou (4) du trou de fixation forme un fraisage du trou de fixation (16).

6. Système selon l'une quelconque des revendications 1 à 5, dans lequel l' ouverture du trou de fixation fileté (18) peut se trouver en position asymétrique par rapport à l'avant-trou (4).

7. Système selon l'une quelconque des revendications 1 à 6, dans lequel la vis de fixation (8) comprend une extrémité proximale (9) avec une contrepartie permettant l'adaptation d'un outil à celle-ci.

8. Système selon la revendication 7, dans lequel une tête de vis (11) ayant un diamètre plus grand que le diamètre extérieur maximum de la section filetée (12) se trouve à l'extrémité proximale (9).

9. Système selon la revendication 8, dans lequel le rapport entre le diamètre de la tête de vis (11) et le diamètre extérieur de la section filetée (12) va de 10/9 à 10/7.

10. Système selon l'une quelconque des revendications 7 ou 8, dans lequel l'avant-trou (4) forme un fraisage (5) et la tête (11) de la vis de fixation est conçue relativement à l'avant-trou (4) de telle sorte qu'elle loge en dessous du niveau de la face supérieure de la plaque.

11. Système selon l'une quelconque des revendications 1 à 7, dans lequel la vis de fixation (8) est une vis sans tête et la section filetée de la vis (12) se prolonge jusqu'à l'extrémité proximale (9).

12. Système selon l'une quelconque des revendications 1 à 11, dans lequel le filetage de la section filetée (12) de la vis de fixation est conçu pour changer de telle sorte que la hauteur du filet de la vis (13) soit plus grande dans la section proche de l'extrémité distale (10) que dans la section plus éloignée de l'extrémité distale.

13. Système selon l'une quelconque des revendications 1 à 12, dans lequel l'angle du trou de fixation fileté (18) peut être choisi pour former un angle différent de l'angle droit à la fois dans le sens longitudinal (L) et dans le sens latéral (W) de la plaque de fixation (1).
